# EUROPEAN PATENT SPECIFICATION

(11) **EP 4 491 205 B1**
(45) Date of publication and mention of the grant of the patent: **28.01.2026**
(21) Application number: 24187008.8
(22) Date of filing: 08.07.2024
(51) Int. Cl.: A61M 3/02, A61B 1/12

(54) **A SYSTEM FOR HEATING AND SUPPLYING A FLUID TO BE SPRAYED INTO AN AINTRACORPOREAL CAVITY**
SYSTEM ZUM ERWÄRMEN UND ZUFÜHREN EINES FLUIDS ZUM BESPRÜHEN IN EINEN INTRACORPORELLEN HOHLRAUM
SYSTÈME DE CHAUFFAGE ET D'ALIMENTATION D'UN FLUIDE À PULVÉRISER DANS UNE CAVITÉ INTRACORPORELLE

(30) Priority: 12.07.2023 IT 202300014529
(43) Date of publication of application: 15.01.2025
(73) Proprietor: Essepi S.r.l. Service and Partners, 00123 Roma (RM) (IT)
(72) Inventor: BIANCOMONTE, Diego, ROMA (RM) (IT); PARLÀ, Domenico, ROMA (RM) (IT); MANTOVANI, Matteo, CARPI (MO) (IT)
(74) Representative: Dall'Olio, Christian

(56) References cited:
- EP-A2- 0 800 835
- EP-B1- 0 292 076
- WO-A1-92/17040
- CN-U- 205 126 983
- US-A- 5 199 604

## Description

### Technical field

The present invention concerns the technical sector relative to instruments and devices for medical use utilised for carrying out clinical-surgical procedures in an intracorporeal cavities of the human body which are reached via natural orifices or special openings, previously made, in proximity of the cavities.

In the clinical sector, the use of endoscopic instruments is made which are introduced into the human body through the natural orifices or special openings in order to reach the intracorporeal area on which a diagnosis is to be carried out or a treatment or a surgical operation is to be performed.

Special channels are present internally of endoscopes for the passage of instruments suitable for carrying out surgical or diagnostic operations, of viewing instruments, as well as for the passage of a saline fluid utilised for spraying the cavity or site on which the diagnostic, therapeutic or surgical operations are to be carried out.

In particular, the present invention concerns a system for heating and supplying a fluid to be sprayed into an intracorporeal cavity which, once heated, is to be supplied via a channel or conduit of the endoscopic instrument which is used in clinical-surgical procedures, such as, for example laparoscopy, laparotomy, hysteroscopy, endoscopy, arthroscopy, endourology.

According to the type of intervention or action to be carried out, the cavity or area reached by the endoscopic instrument must be appropriately sprayed with a saline fluid with the purpose of washing, in order to keep the visual field of the operator clear, for example by removing corporeal fluids that may be present, or for dilating the tissues thus to enable the carrying out of the surgical procedure required, as well as preventing the patient's body temperature from excessively dropping, leading to hypothermia.

A first factor that needs taking into consideration is constituted by the pressure with which the fluid is sprayed and reaches the cavity or area of interest.

A second important factor relates to the temperature of the fluid to be sprayed, in fact an excessively low temperature not only creates problems of hypothermia but can have a negative impact on other haemodynamic parameters of the patient.

For example, in endourological surgery the method of transurethral resection of the prostate (TURP) represents the surgical treatment of first choice in practically all patients affected by benign prostatic hypertrophy that is no longer responsive to medical treatment.

Technically, the operation is made possible by a continuous washing of the cavities with solutions of mannitol or saline, with the inlet of washing liquid and exit thereof through the actual resectoscope.

Also in the case of transurethral resection of the bladder a spraying solution is utilised during the operation so as to distend the bladder.

The spraying of the cavities subject to operation by fluids not appropriately controlled in terms of the spraying pressure thereof can create hyperdistensions of the cavities, and if they have an excessively low temperature with respect to the body temperature they can cause hypothermia with post-operative risks.

In this regard, it has been demonstrated how the use of spraying fluids not appropriately heated to an adequate temperature during transurethral prostatectomy operations can have impacts on the haemodynamic variables of the patients, such as for example a lowering of the arterial pressure, a systemic vascular resistance, bradycardia and lowering of the stroke volume.

### Description of the prior art

A first known system used for heating a fluid which is to be sprayed into an intracorporeal cavity by means of an endoscopic instrument comprises use of a stator, positioned at a certain height with respect to the operating site, on which a consumable bag made of a plastic material is positioned and hooked, for example made of PVC, defining internally thereof a containing chamber of a saline fluid and having an outlet conduit of the fluid.

The stator, powered by an electrical current, heats the bag and thus the fluid contained internally thereof.

The bag, via the outlet conduit, is set in communication with a supply channel that is connected to the endoscopic instrument.

The supply of the heated fluid to the endoscopic instrument, in this case, takes place by force of gravity.

The height at which the stator is positioned with respect to the operating site will then determine the supply pressure value of the fluid.

A system of this type presents various drawbacks.

Primarily, the delay time, i.e. the waiting time for heating the fluid, before the fluid can be sprayed, is quite long, and this circumstance can lengthen the operating times.

Secondly, there is no possibility of adequately regulating the supply pressure of the fluid, as it depends only on the height at which the stator is positioned, and, further, with the reduction of the content of the fluid internally of the bag, the pressure will undergo variations.

This negatively affects the possibility of maintaining a constant pressure value during the whole time during which the fluid is sprayed into the cavity, and can cause undesired fluctuations of the fluid flow, as well as the presence of air bubbles internally thereof.

Further, the heating of the fluid internal of the bag is not uniform, and therefore the fluid can reach the cavity at an inappropriate temperature, and thus not completely heated to the appropriate temperature, causing the issues as previously evidenced, or at an excessive temperature, a circumstance that can lead to the risk of scalding in the patient.

Lastly, once the fluid in the bag has run out, it will have to be replaced with another bag with a relative fluid to be heated, leading to a discontinuity in the operations.

Another type of known system for carrying out the heating and supply of a fluid to be sprayed into an intracorporeal cavity is constituted by a heating oven, for example by means of infrared rays or electrical resistances, internally of which a consumable bag made of a plastic material is positioned, for example made of PVC, defining internally thereof a containing chamber of a saline fluid and having an outlet conduit of the fluid, which is connected to a supply channel communicating with the endoscopic instrument.

An overpressure is created in the oven, by injection of pressurised air, so as to compress the bag in such a way as to supply the heated fluid to the endoscopic instrument for subsequent spraying into the intracorporeal cavity subjected to the treatment or to the surgical operation.

With this system too some drawbacks are evident, linked to a non-uniform heating of the fluid present in the chamber of the bag, concerning both the onset of fluctuations in the pumping of the heated fluid, with the formation of air bubbles in the fluid before the spraying thereof into the cavities.

Further, this system too cannot guarantee a continuity in the fluid spraying operations, as the bag, once emptied of the fluid, must be removed from the oven and replaced with a new bag full of fluid.

CN205126983U relates to a system for heating and supplying a fluid to be sprayed into an intracorporeal cavity, namely a thoracoscopic surgery flushing device.

EP0292076 A1 relates to a system suitable for heating blood for transfusion.

### Summary of the invention

The aim of the present invention is therefore to provide a novel system for heating and supplying a fluid to be sprayed into an intracorporeal cavity able to obviate the drawbacks present in the prior art and mentioned in the foregoing.

In particular, the aim of the invention is to provide a new system able to carry out a uniform heating of the fluid and a supply at pressure of the fluid which is controlled and substantially free of fluctuations.

A further aim of the invention is to provide a new system which enables a continuous supply of the heated fluid as well as a drastic reduction of any latency in times, i.e. the waiting times before proceeding with the spraying of the fluid into the intracorporeal cavity.

The above aims are attained according to the claims.

### Brief description of the drawings

The characteristics of preferred, but not exclusive, embodiments of the system for heating and supplying a fluid to be sprayed into an intracorporeal cavity of the present invention will be described in the following with reference to the appended tables of drawings, in which:
- figure 1 illustrates, is a schematic frontal view, a first significant component of the system of the invention, for containing and passage of the fluid to be sprayed into an intracorporeal cavity;
- figure 2 illustrates the component of figure 1 associated to other components of the system of the invention, for supplying at pressure the fluid to be sprayed into an intracorporeal cavity;
- figure 3 illustrates, schematically and in a partial perspective view, other significant components of the system for heating the fluid to be sprayed into an intracorporeal cavity;
- figure 4 illustrates, in a schematic perspective view one of the components of the system used for heating the fluid to be sprayed into an intracorporeal cavity;
- figure 5 illustrates a detail of a component of the system for heating the fluid to be sprayed into an intracorporeal cavity;

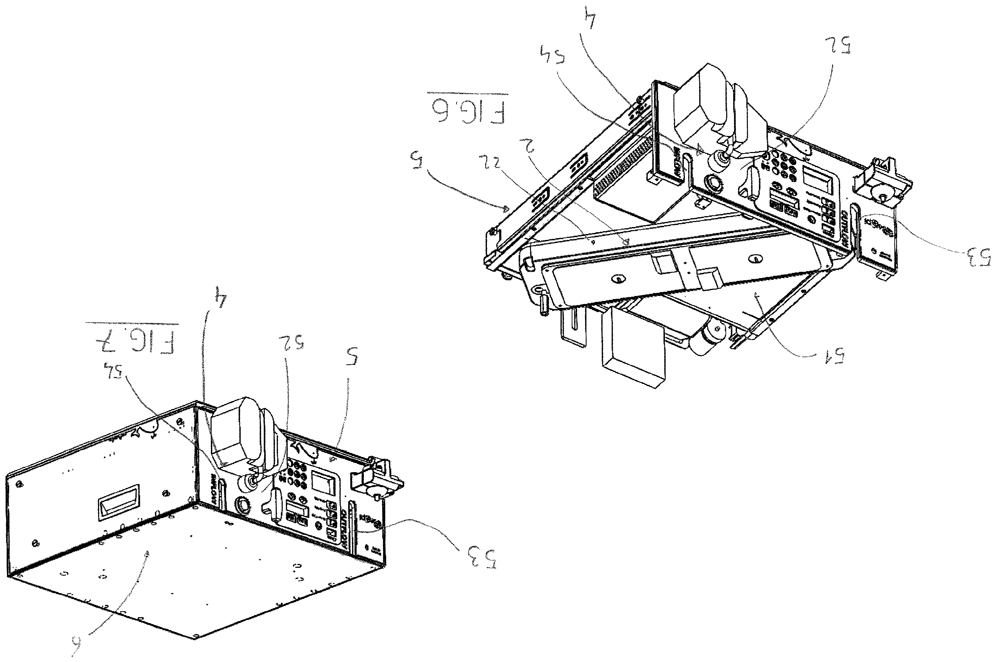

- figures 6 and 7 illustrate, in respective schematic perspective views, a possible preferred modality of arrangement and application and the components of the system;
- figure 8 illustrates, in a partial schematic view, other component details of the system of the invention utilisable in the application illustrated in the figures 6 and 7;
- figure 9 illustrates, in a frontal view, the component of figure 1 according to a particular embodiment;
- figure 10 is a graph in which various trend curves are illustrated relating to the supply pressure of a fluid to be sprayed into an intracorporeal cavity, in which: the fluid is supplied by means of a peristaltic pump through a supply conduit (curve C1), the fluid is supplied by means of the component of the system of the invention of figure 1, in a first modality of actuation (curve C2) and wherein the fluid is supplied by means of the component of the system of the invention of figure 1 and on the basis of a preferred embodiment of the system.

### Description of the preferred embodiments of the invention

The above-described figures illustrate the various significant components of the system for heating and supplying a fluid, for example a saline or mannitol solution, or any other type of compatible fluid, to be sprayed into an intracorporeal cavity, described in the present invention.

The system of the invention, in its main and essential components, comprises: a bag (1) made of a plastic material that is deformable following application of heat for enabling an expansion of the bag, a pair of heating plates (2, 3) and a peristaltic pump (4), which are configured and arranged in the following way.

The bag made of a deformable plastic material, for example PVC, comprises a first inlet conduit (11) for inlet into the bag (1) of a fluid to be sprayed into an intracorporeal cavity, and a second outlet conduit (12) for outlet of the fluid from the bag (1)

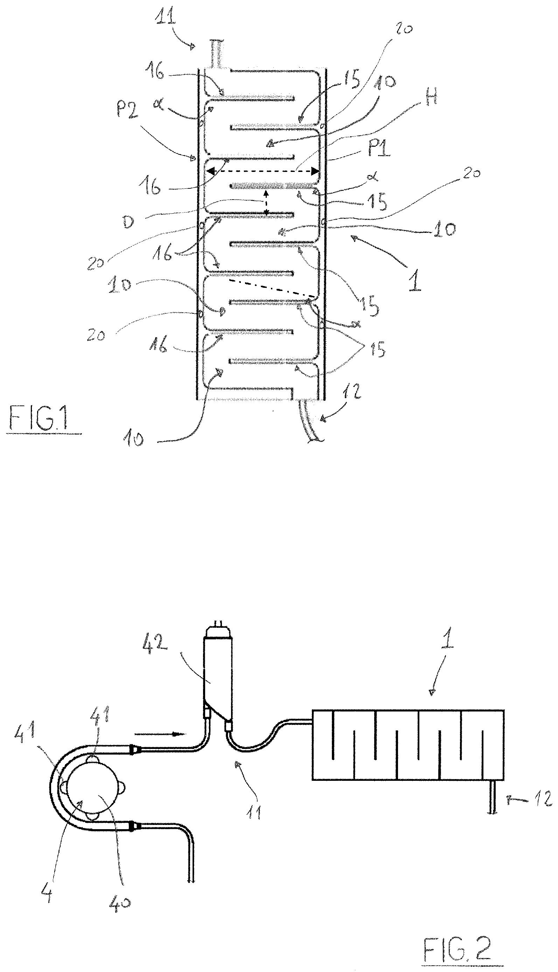

The bag (1) (see for example figure 1 and figure 2, or figure 9) is conformed in such a way as to comprise internally thereof a first series of partition walls (15) which originate from a first lateral edge (P1) of the bag (1) and a second series of partition walls (16) which originate from a second lateral edge (P2) of the bag (1), wherein the first series of partition walls (15) is intercalated with the second series of partition walls (16) in such a way as to identify, internally of the bag (1), a fluid conveying path in the form of a serpentine (10) which extends from the first inlet conduit (11) to the second outlet conduit (12).

The first inlet conduit (11) of the bag (1) is configured to be connected to a supply source of fluid to be sprayed into an intracorporeal cavity in such a way that the fluid can enter the bag (1) and flow along the fluid conveying path in the form of a serpentine (10) up to the second outlet conduit (12).

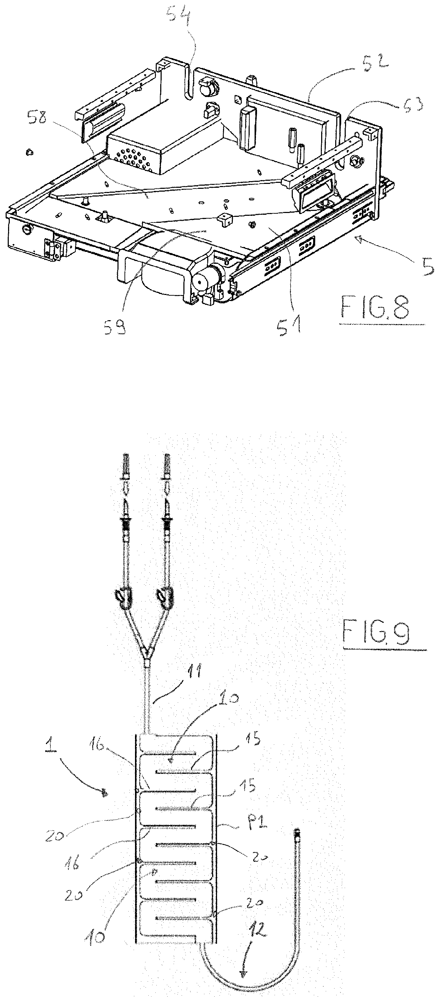

For example, in a preferred embodiment that is not exclusive, the first inlet conduit (11) can comprise a bifurcation in such a way as to be connectable to two distinct fluid supply pipes communicating with two distinct fluid supply sources to be sprayed into an intracorporeal cavity (see for example figure 9).

In this way, the system of the invention can guarantee continuity of fluid supply to inside the bag, as, when a first supply source of fluid is exhausted, the bag can receive the fluid from a second supply source of fluid.

The second outlet conduit (12) is configured to be connected to an endoscopic instrument suitable for being able to carry out the diagnostic or surgical operations required, so that the fluid in outlet from the second outlet conduit is sent into a channel present internally of the endoscopic instrument in order to be sprayed into the intracorporeal cavity, or site in which the endoscopic instrument is to carry out diagnostic and/or surgical operations.

The pair of heating plates (2, 3) are configured and arranged in such a way as to be able to receive between them the bag (1) and, when activated, to heat the bag (1) and the contents thereof.

In particular, the pair of heating plates (2, 3) are conformed and reciprocally arranged in such a way as to define between them a space for the bag (1) which enables an expansion of the bag (1) when the bag is heated.

For example, the heating plates can be of the electrical resistance type, as provided with electric resistors or serpentines.

Use is also possible of heating plates using another type of heating.

The peristaltic pump (4) is arranged in such a way as to establish a fluid pumping flow coming from the supply source in such a way that the fluid flows along the fluid conveying path in the form of a serpentine (10) acquiring heat from the pair of heating plates (2, 3) so that the heated fluid is pumped through the second outlet conduit (12) to the endoscopic instrument in order to be sprayed into an intracorporeal cavity.

With this peculiarity, with the system of the invention it is possible to pump, in outlet from the bag (1), through the second outlet conduit (12), a fluid that has already been heated as the fluid arriving from the supply source of the fluid has crossed the entire fluid conveying path in the form of a serpentine (10) internally of the bag (1) and has acquired heat from the pair of heating plates (2, 3).

For example, the pair of heating plates (2, 3) are configured and activatable so as to heat the bag (1) to a temperature which can be comprised in a temperature range of 45°-60°C.

In turn, the peristaltic pump (4) can be activatable to offer a pumping pressure which can vary within a range from 50 to 500 mbar.

Owing to the special inner conformation of the bag (1), the pumping pressure of the fluid is regularised during its flow along the fluid conveying path in the form of a serpentine (10) in the bag (1), as the bag (1) deforms and expands, due to the heat received from the pair of heating plates (2, 3), thus behaving as a sort of "damper", i.e. an absorber of any eventual fluctuations present in the flow.

In other words, the presence of any air bubbles internally of the fluid flow, which might occur due to the type of pumping of the peristaltic pump, are dilutated and eliminated due to the increase in volume of the bag, which enables a minimising of any detachment of the confined flow running into the bag.

This allows a reduction of any turbulence during the circulation of the fluid in the bag, further preventing an onset of "hot spots", i.e. areas in which the temperature of the fluid rises excessively, and thus enables avoiding, or at least significantly reducing, any risks of scalsing to the patient.

Further, the fluid arriving from the supply source of the fluid, once it has entered the bag through the first inlet conduit, and while crossing the fluid conveying path in the form of a serpentine (10), is uniformly heated by the heat supplied by the pair of heating plates, and then can exit from the bag, through the second outlet conduit (12), at a suitable temperature for being sprayed into an intracorporeal cavity via a suitable endoscopic instrument connected to the second outlet conduit (12) of the bag (1).

Other further advantageous characteristics of the system of the invention are described in the following.

The partition walls of the first series of partition walls (15) present internally of the bag (1) are arranged with respect to the first lateral edge (P1) of the bag (1) in such a way as to form, with respect to the first lateral edge (P1), an angle (α) comprised between 90° and 150° (see the broken line in figure 1) and wherein the partition walls of the second series of partition walls (16) present internally of the bag (1) are arranged, with respect to the second lateral edge (P2) of the bag (1), in such a way as to form an angle comprised between 90° and 150° with respect to the second lateral wall.

In a further preferred aspect, the ratio between the distance (H) (indicated in figure 1) between the first lateral edge (P1) and the second lateral edge (P2) of the bag (1), and the distance (D) (indicated in figure 1) beween two adjacent partition walls of the first (15) and second (16) series of partition walls is comprised in the range of 3.5 - 10.

The peristaltic pump (4) comprises a rotor (40) peripherally having a series of lobes (41), with the peristaltic pump (4) arranged so that the lobes (41), when the rotor (40) is activated in rotation, act at the inlet conduit (11) or the outlet conduit (12) of the bag (1) so as to establish a pumping flow of the fluid coming from the supply source in such a way that the fluid flows along the fluid conveying path in the form of a serpentine (10), acquiring heat from the pair of heating plates (2, 3) so that the heated fluid is pumped through the second outlet conduit (12) to the endoscopic instrument in order to be sprayed into an intracorporeal cavity.

In a further particularly preferred aspect, the peristaltic pump (4) is arranged in such a way that the lobes (41), when the rotor (40) is activated in rotation, act at the inlet conduit (11) of the bag (1), with the system comprising an expansion chamber (42) along the first inlet conduit (11), in a position interposed between the bag (1) and the portion of the inlet conduit (11) on which the lobes (41) of the rotor (40) act (see for example the circuit diagram reported in figure 2).

In this way, the fluid pumped by the peristaltic pump (4) is first supplied into the expansion chamber (42), partially filling it, before being pumped into the bag (1) and crossing the fluid conveying path in the form of a serpentine (10), enabling a further reduction of the onset of any fluctuations of the fluid flow during the circulation thereof internally of the bag (1).

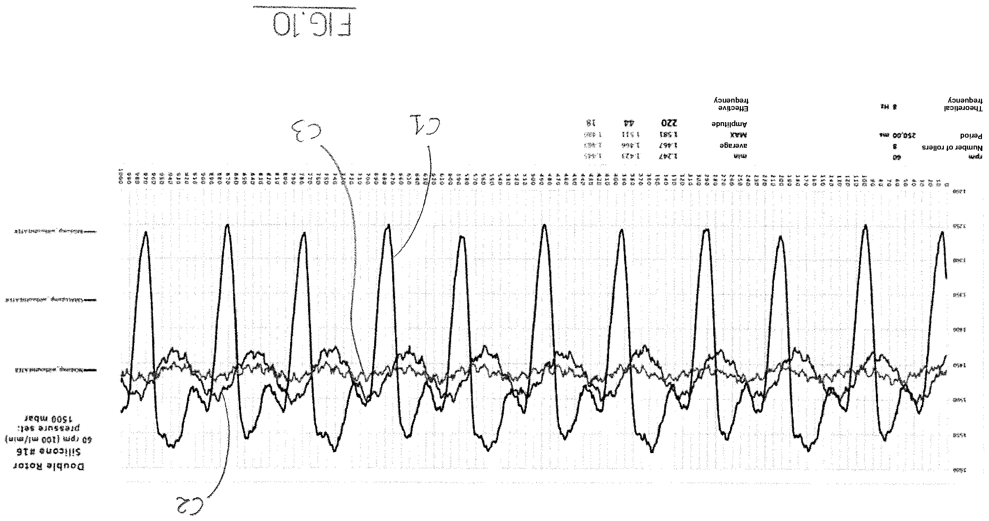

In this matter, figure 10 includes the graphs relating to the progress of the fluid flow in three distinct situations.

Curve C1 indicates the progress of the fluid flow when it is pumped from a peristaltic pump through a normal conduit.

Curve C2 on the other hand indicates the progress of the fluid flow when it is pumped to cross the bag of the invention, with no heating.

It can be noted that the curve C2 has fewer fluctuations than curve C1 owing to the particular inner conformation of the bag (1) which includes the flow conveying path in the form of a serpentine (10).

Curve C3 indicates the progress of the fluid flow when it is pumped to cross the bag of the invention, with no heating, with the presence of the expansion chamber upstream of the bag.

It can be noted that curve C3 has fewer fluctuations than curve C2, owing to the presence of the expansion chamber upstream of the bag.

In the cases of curve C2 and curve C3, the expansion of the bag, with the relative heating, will enable having a further reduction of the fluctuations present in the fluid during the circulation thereof internally of the bag along the fluid conveying path having the serpentine shape.

On the basis of the preferred but not exclusive embodiment, illustrated in the figures, the system of the invention can be made and configured in such a way that a first heating plate (2) of the pair of heating plates (2, 3) is arranged and mounted on a first structure (5) having a drawer or slide shape and wherein the second heating plate (3) of the pair of heating plates (2, 3) is arranged and mounted on a second structure (6) having a container shape.

In particular, the second structure (6) having a container shape is conformed in such a way as to comprise internally thereof a housing, accessible from outside, having a shape such as to slidably receive the first structure (5) having a drawer or slide shape in such a way that the first structure (5) having a drawer or slide shape is placed in a position such that the first heating plate (2) is arranged facing the second heating plate (3) so as to close between them the bag (1) arranged and positioned on the first heating plate (2).

The bag (1) preferably comprises respective slots (20) on the relative first and second lateral edges (P1, P2) with the first heating plate (2) comprising, peripherally on relative lateral edges, hook elements (22) conformed so as to be inserted in the slots (20) of the bag (1) in order to ensure the blocking of the bag (1) on the first heating plate (2).

The first structure (5) having a drawer or slide shape comprises a base wall (51), on which the first heating plate (2) is mounted and arranged, and a front wall (52) arranged vertically at a first lateral edge of the base wall (51), wherein, when the first structure (5) having a drawer or slide shape is inserted in the housing of the second structure (6) having a container shape, the front wall (52) remains directly accessible from outside.

In this way, it is possible to position the bag (1) on the first heating plate, and block it by coupling and insertion of the hook elements (22) in the slots (20) and then insert the first structure (5) into the housing of the second structure (6).

In this matter, the front wall (52) of the first structure (5) comprises a first aperture (53), having a shape suitable for allowing passage of the first inlet conduit (11) of the bag (1) in such a way that the first inlet conduit can be connected to the supply source of the fluid, and a second aperture (54), having a shape suitable for allowing passage of the second outlet conduit (12) of the bag (1) in such a way that the second outlet conduit can be connected to an endoscopic instrument.

The rotor (40) of the peristaltic pump (4) is mounted on the front wall (52) in a position such as to remain positioned internally of the second structure (6) in the form of a container when the first structure (5) having a drawer or slide shape is inserted in the housing of the second structure (6) having the shape of a container.

Further, in this particular embodiment, the system of the invention can further comprise also at least an inclined separating wall (58), which is arranged in the first structure (5) having a drawer or slide shape, below the first heating plate (2), and which is configured to receive any leaks of fluid exiting from the bag (1) and to direct the leaks of fluid towards a collection tray (59) also arranged in the first structure (5) having a drawer or slide shape.

The system can advantageously also comprise a moisture detector, positioned in the first structure (5) in proximity or at the inclined separating wall (58) or at the collection tray (59), in such a way as to detect any leaks of fluid from the bag, and thus enable the extraction of the first structure (5) from the second structure (6) and the removal of the damaged bag.

Another further preferred aspect of the system, in the particular embodiment described in the foregoing, consists in the arrangement of the pair of heating plates which can be arranged diagonally in the first structure (5) and in the second structure (6), with an angle of inclination with respect to an axial axis comprised in a range between 30° and 70°.

In this way, it is possible to use heating plates of larger dimensions with respect to the case of an arrangement thereof in an axial direction.

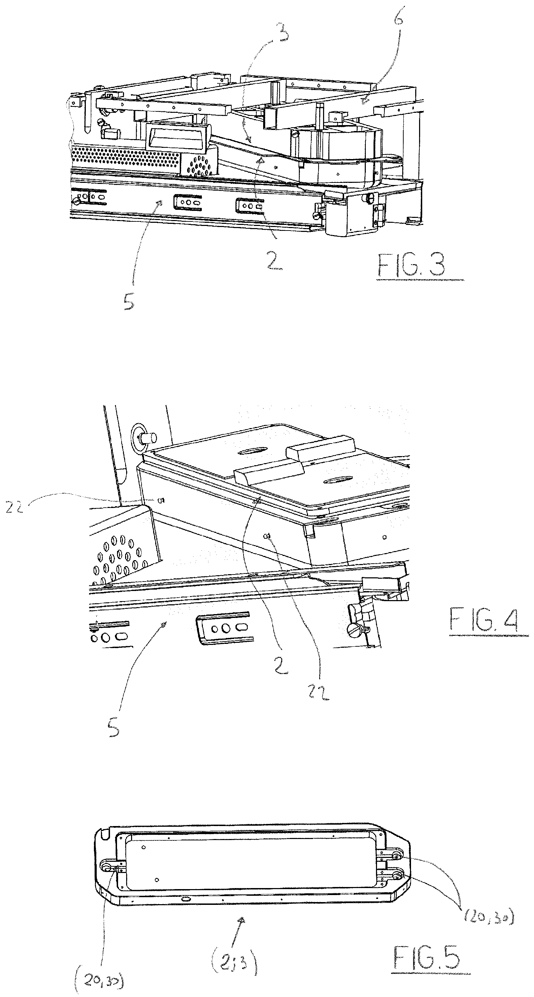

The pair of heating plates (2, 3) comprise temperature detectors (20, 30) arranged and configured to detect the heating temperature of the bag (1) (see for example figure 5), for example constituted by thermocouples.

The temperature detectors (20, 30) are interfaced with a command and control unit (not illustrated, and arranged either on the first structure or on the second structure), arranged in such a way as to be able to intervene on the heating plates in order to increase or reduce the heating temperature thereof according to needs.

Further, the system can also comprise at least a pressure detector (not illustrated), arranged in such a way as to detect the pumping pressure of the flow of fluid which crosses the flow conveying path in the form of a serpentine (10) of the bag (1).

The pressure detector is also interfaced with the command and control unit in order to enable a regulating of the functioning of the peristaltic pump.

## Claims

1. A system for heating and supplying a fluid to be sprayed into an intracorporeal cavity, comprising:
a bag (1) made of a plastic material that is deformable following application of heat for enabling an expansion of the bag (1), the bag comprising a first inlet conduit (11) for inlet into the bag (1) of a fluid to be sprayed into an intracorporeal cavity, and a second outlet conduit (12) for outlet of the fluid from the bag (1), wherein the bag (1) is conformed in such a way as to comprise internally thereof a first series of partition walls (15) which originate from a first lateral edge (P1) of the bag (1) and a second series of partition walls (16) which originate from a second lateral edge (P2) of the bag (1), wherein the first series of partition walls (15) is intercalated with the second series of partition walls (16) in such a way as to identify, internally of the bag (1), a fluid conveying path in the form of a serpentine (10) which extends from the first inlet conduit (11) to the second outlet conduit (12);
a pair of heating plates (2, 3) which are configured and arranged in such a way as to be able to receive between them the bag (1) and, when activated, to heat the bag (1) and the contents thereof, wherein the pair of heating plates (2, 3) are conformed and reciprocally arranged in such a way as to define between them a space for the bag (1) which enables an expansion of the bag (1) when the bag is heated;
a peristaltic pump (4);
wherein the first inlet conduit (11) of the bag (1) is configured to be connected to a supply source of fluid to be sprayed into an intracorporeal cavity in such a way that the fluid can enter the bag (1) and flow along the fluid conveying path in the form of a serpentine (10) up to the second outlet conduit (12);
wherein the second outlet conduit (12) is configured to be connected to an endoscopic instrument;
and wherein the peristaltic pump (4) is arranged in such a way as to establish a pumping flow of the fluid coming from the supply source in such a way that the fluid flows along the fluid conveying path in the form of a serpentine (10) acquiring heat from the pair of heating plates (2, 3) so that the heated fluid is pumped through the second outlet conduit (12) to the endoscopic instrument in order to be sprayed into an intracorporeal cavity.

2. The system as claimed in claim 1, wherein the partition walls of the first series of partition walls (15) present internally of the bag (1) are arranged with respect to the first lateral edge (P1) of the bag (1) in such a way as to form, with respect to the first lateral edge (P1), an angle (α) comprised between 90° and 150° and wherein the partition walls of the second series of partition walls (16) present internally of the bag (1) are arranged, with respect to the second lateral edge (P2) of the bag (1), in such a way as to form an angle comprised between 90° and 150° with respect to the second lateral wall.

3. The system as claimed in any one of the preceding claims, wherein the ratio between the distance (H) between the first lateral edge (P1) and the second lateral edge (P2) of the bag (1), and the distance (D) beween two adjacent partition walls of the first (15) and second (16) series of partition walls is comprised in the range of 3.5 - 10.

4. The system as claimed in any one of the preceding claims, wherein the peristaltic pump (4) comprises a rotor (40) peripherally having a series of lobes (41), and wherein the peristaltic pump (4) is arranged so that the lobes (41), when the rotor (40) is activated in rotation, act at the inlet conduit (11) or the outlet conduit (12) of the bag (1) so as to establish a pumping flow of the fluid coming from the supply source in such a way that the fluid flows along the fluid conveying path in the form of a serpentine (10), acquiring heat from the pair of heating plates (2, 3) so that the heated fluid is pumped through the second outlet conduit (12) to the endoscopic instrument in order to be sprayed into an intracorporeal cavity.

5. The system as claimed in claim 4, wherein the peristaltic pump (4) is arranged in such a way that the lobes (41), when the rotor (40) is activated in rotation, act at the inlet conduit (11) of the bag (1) and wherein the system (100) comprises an expansion chamber (42) along the first inlet conduit (11), in a position interposed between the bag (1) and the portion of the inlet conduit (11) on which the lobes (41) of the rotor (40) act, in such a way that the fluid is supplied and partially fills the expansion chamber (41) before being pumped into the bag (1) and crossing the fluid conveying path in the form of a serpentine (10).

6. The system as claimed in any one of the preceding claims, wherein a first heating plate (2) of the pair of heating plates (2, 3) is arranged and mounted on a first structure (5) having a drawer or slide shape and wherein the second heating plate (3) of the pair of heating plates (2, 3) is arranged and mounted on a second structure (6) having a container shape conformed in such a way as to comprise internally thereof a housing, accessible from outside, having a shape such as to slidably receive the first structure (5) having a drawer or slide shape in such a way that the first structure (5) having a drawer or slide shape is placed in a position such that the first heating plate (2) is arranged facing the second heating plate (3) so as to close between them the bag (1) arranged and positioned on the first heating plate (2).

7. The system as claimed in claim 6, wherein the bag (1) comprises respective slots (20) on the relative first and second lateral edges (P1, P2) and wherein the first heating plate (2) comprises, peripherally on relative lateral edges, hook elements (22) conformed so as to be inserted in the slots (20) of the bag (1) in order to ensure the blocking of the bag (1) on the first heating plate (2).

8. The system as claimed in claim 6, wherein the first structure (5) having a drawer or slide shape comprises a base wall (51), on which the first heating plate (2) is mounted and arranged, and a front wall (52) arranged vertically at a first lateral edge of the base wall (51), wherein, when the first structure (5) having a drawer or slide shape is inserted in the housing of the second structure (6) having a container shape, the front wall (52) remains directly accessible from outside, and wherein the front wall (52) comprises a first aperture (53), having a shape suitable for allowing passage of the first inlet conduit (11) of the bag (1) in such a way that the first inlet conduit can be connected to the supply source of the fluid, and a second aperture (54), having a shape suitable for allowing passage of the second outlet conduit (12) of the bag (1) in such a way that the second outlet conduit can be connected to an endoscopic instrument, and wherein the rotor (40) of the peristaltic pump (4) is mounted on the front wall (52) in a position such as to remain positioned internally of the second structure (6) in the form of a container when the first structure (5) having a drawer or slide shape is inserted in the housing of the second structure (6) in the shape of a container.

9. The system as claimed in any one of claims from 6 to 8, comprising at least an inclined separating wall (58), arranged in the first structure (5) having a drawer or slide shape, below the first heating plate (2), configured to receive any leaks of fluid exiting from the bag (1) and to direct the leaks of fluid towards a collection tray (59) also arranged in the first structure (5) having a drawer or slide shape.

10. The system of any one of the preceding claims, wherein the pair of heating plates (2, 3) comprise temperature detectors (20, 30) arranged and configured to detect the heating temperature of the bag (1).

11. The system of any one of the preceding claims, comprising at least a pressure detector, arranged in such a way as to detect the pumping pressure of the flow of fluid which crosses the flow conveying path in the form of a serpentine (10) of the bag (1).

## Patentansprüche

1. System zum Erwärmen und Zuführen eines Fluids, das in eine Körperhöhle gesprüht werden soll, umfassend:
einen Beutel (1) aus einem Kunststoffmaterial, das nach Anwendung von Wärme verformbar ist, um eine Ausdehnung des Beutels (1) zu ermöglichen, wobei der Beutel eine erste Einlassleitung (11) für den Einlass eines in eine Körperhöhle zu spritzenden Fluids in den Beutel (1) und eine zweite Auslassleitung (12) für den Auslass des Fluids aus dem Beutel (1) umfasst, wobei der Beutel (1) so geformt ist, dass er in seinem Inneren eine erste Reihe von Trennwänden (15), die von einer ersten Seitenkante (P1) des Beutels (1) ausgehen, und eine zweite Reihe von Trennwänden (16), die von einer zweiten Seitenkante (P2) des Beutels (1) ausgehen, umfasst, wobei die erste Reihe von Trennwänden (15) mit der zweiten Reihe von Trennwänden (16) so verschachtelt ist, dass sie im Inneren des Beutels (1) einen Flüssigkeitstransportweg in Form einer Serpentine (10) bilden, die sich von der ersten Einlassleitung (11) zur zweiten Auslassleitung (12) erstreckt;
ein Paar Heizplatten (2, 3), die so konfiguriert und angeordnet sind, dass sie den Beutel (1) zwischen sich aufnehmen können und bei Aktivierung den Beutel (1) und dessen Inhalt erwärmen, wobei das Paar Heizplatten (2, 3) so geformt und gegenseitig angeordnet ist, dass sie zwischen sich einen Raum für den Beutel (1) definieren, der eine Ausdehnung des Beutels (1) ermöglicht, wenn der Beutel erwärmt wird;
eine Peristaltikpumpe (4);
wobei die erste Einlassleitung (11) des Beutels (1) so konfiguriert ist, dass sie mit einer Versorgungsquelle für Flüssigkeit verbunden werden kann, die in eine Körperhöhle gesprüht werden soll, sodass die Flüssigkeit in den Beutel (1) eintreten und entlang des Flüssigkeitstransportwegs in Form einer Serpentine (10) bis zur zweiten Auslassleitung (12) fließen kann;
wobei die zweite Auslassleitung (12) so konfiguriert ist, dass sie mit einem endoskopischen Instrument verbunden werden kann;
und wobei die Peristaltikpumpe (4) so angeordnet ist, dass sie einen Pumpfluss der aus der Versorgungsquelle kommenden Flüssigkeit erzeugt, sodass die Flüssigkeit entlang des Flüssigkeitstransportwegs in Form einer Serpentine (10) fließt und dabei Wärme von dem Paar Heizplatten (2, 3) Wärme aufnimmt, so dass das erwärmte Fluid durch die zweite Auslassleitung (12) zum endoskopischen Instrument gepumpt wird, um in eine intrakorporale Höhle gesprüht zu werden.

2. Das System nach Anspruch 1, wobei die Trennwände der ersten Reihe von Trennwänden (15), die sich im Inneren des Beutels (1) befinden, in Bezug auf die erste Seitenkante (P1) des Beutels (1) so angeordnet sind, dass sie in Bezug auf die erste Seitenkante (P1) einen Winkel (α) zwischen 90° und 150° bilden, und wobei die Trennwände der zweiten Reihe von Trennwänden (16), die sich im Inneren des Beutels (1) befinden, in Bezug auf die zweite Seitenkante (P2) des Beutels (1) so angeordnet sind, dass sie einen Winkel zwischen 90° und 150° in Bezug auf die zweite Seitenwand bilden.

3. Das System nach einem der vorstehenden Ansprüche, wobei das Verhältnis zwischen dem Abstand (H) zwischen der ersten Seitenkante (P1) und der zweiten Seitenkante (P2) des Beutels (1) und dem Abstand (D) zwischen zwei benachbarten Trennwänden der ersten (15) und zweiten (16) Reihe von Trennwänden im Bereich von 3,5 bis 10 liegt.

4. Das System nach einem der vorstehenden Ansprüche, wobei die Peristaltikpumpe (4) einen Rotor (40) mit einer Reihe von Nocken (41) am Umfang umfasst und wobei die Peristaltikpumpe (4) so angeordnet ist, dass die Nocken (41) , wenn der Rotor (40) in Drehung versetzt wird, auf die Einlassleitung (11) oder die Auslassleitung (12) des Beutels (1) einwirken, um einen Pumpfluss des aus der Versorgungsquelle kommenden Fluids in der Weise herzustellen, dass das Fluid entlang des Fluidförderwegs in Form einer Serpentine (10) fließt und dabei Wärme von dem Paar Heizplatten (2, 3) Wärme aufnimmt, so dass das erwärmte Fluid durch die zweite Auslassleitung (12) zum endoskopischen Instrument gepumpt wird, um in eine intrakorporale Höhle gesprüht zu werden.

5. Das System nach Anspruch 4, wobei die Peristaltikpumpe (4) so angeordnet ist, dass die Lappen (41), wenn der Rotor (40) in Drehung versetzt wird, auf die Einlassleitung (11) des Beutels (1) einwirken, und wobei das System (100) eine Expansionskammer (42) entlang der ersten Einlassleitung (11) umfasst, in einer Position zwischen dem Beutel (1) und dem Abschnitt der Einlassleitung (11), auf den die Lappen (41) des Rotors (40) einwirken, so dass die Flüssigkeit zugeführt wird und die Expansionskammer (41) teilweise füllt, bevor sie in den Beutel (1) gepumpt wird und den Flüssigkeitstransportweg in Form einer Serpentine (10) durchläuft.

6. Das System nach einem der vorstehenden Ansprüche, wobei eine erste Heizplatte (2) des Paares von Heizplatten (2, 3) auf einer ersten Struktur (5) mit einer Schubladen- oder Schiebeform angeordnet und montiert ist und wobei die zweite Heizplatte (3) des Paares von Heizplatten (2, 3) auf einer zweiten Struktur (6) angeordnet und montiert ist, die eine Behälterform aufweist, die so gestaltet ist, dass sie in ihrem Inneren ein von außen zugängliches Gehäuse umfasst, das so geformt ist, dass es die erste Struktur (5) mit einer Schubladen- oder Schiebeform verschiebbar aufnehmen kann, so dass die erste Struktur (5) mit einer Schubladen- oder Schiebeform in einer Position angeordnet ist, in der die erste Heizplatte (2) der zweiten Heizplatte (3) gegenüberliegt (3) angeordnet ist, um zwischen ihnen den Beutel (1) zu verschließen, der auf der ersten Heizplatte (2) angeordnet und positioniert ist.

7. Das System nach Anspruch 6, wobei der Beutel (1) entsprechende Schlitze (20) an den relativen ersten und zweiten Seitenkanten (P1, P2) aufweist und wobei die erste Heizplatte (2) am Umfang der jeweiligen Seitenkanten Hakenelemente (22) aufweist, die so geformt sind, dass sie in die Schlitze (20) des Beutels (1) eingeführt werden können, um die Arretierung des Beutels (1) auf der ersten Heizplatte (2) sicherzustellen.

8. Das System nach Anspruch 6, wobei die erste Struktur (5) mit einer Schubladen- oder Schiebeform eine Basiswand (51), auf der die erste Heizplatte (2) montiert und angeordnet ist, und eine Vorderwand (52) umfasst, die vertikal an einer ersten Seitenkante der Basiswand angeordnet ist (51) angeordnet ist, wobei, wenn die erste Struktur (5) mit einer Schubladen- oder Schiebeform in das Gehäuse der zweiten Struktur (6) mit einer Behälterform eingesetzt ist, die Vorderwand (52) von außen direkt zugänglich bleibt, und wobei die Vorderwand (52) eine erste Öffnung (53) umfasst, mit einer Form, die geeignet ist, den Durchgang der ersten Einlassleitung (11) des Beutels (1) so zu ermöglichen, dass die erste Einlassleitung mit der Versorgungsquelle des Fluids verbunden werden kann, und eine zweite Öffnung (54) mit einer Form, die geeignet ist, den Durchgang der zweiten Auslassleitung (12) des Beutels (1) so zu ermöglichen, dass die zweite Auslassleitung mit einem endoskopischen Instrument verbunden werden kann, und wobei der Rotor (40) der Peristaltikpumpe (4) an der Vorderwand (52) in einer Position angebracht ist, so dass er innerhalb der zweiten Struktur (6) in Form eines Behälters positioniert bleibt, wenn die erste Struktur (5) mit einer Schubladen- oder Schiebeform in das Gehäuse der zweiten Struktur (6) in Form eines Behälters eingesetzt wird.

9. System nach einem der Ansprüche 6 bis 8, umfassend mindestens eine geneigte Trennwand (58), die in der ersten Struktur (5) in Form einer Schublade oder eines Schiebers unterhalb der ersten Heizplatte (2) angeordnet ist die so konfiguriert ist, dass sie aus dem Beutel (1) austretende Flüssigkeitsleckagen auffängt und die Flüssigkeitsleckagen zu einer ebenfalls in der ersten Struktur (5) mit Schubladen- oder Schiebeform angeordneten Auffangschale (59) leitet.

10. Das System nach einem der vorstehenden Ansprüche, wobei das Paar Heizplatten (2, 3) Temperaturdetektoren (20, 30) umfasst, die so angeordnet und konfiguriert sind, dass sie die Heiztemperatur des Beutels (1) erfassen.

11. Das System nach einem der vorstehenden Ansprüche, umfassend mindestens einen Druckdetektor, der so angeordnet ist, dass er den Pumpdruck des Flüssigkeitsstroms erfasst, der den Flüssigkeitsförderweg in Form einer Serpentine (10) des Beutels (1) durchläuft.

## Revendications

1. Système pour chauffer et fournir un fluide à pulvériser dans une cavité intracorporelle, comprenant :
un sac (1) en matière plastique déformable sous l'effet de la chaleur afin de permettre son expansion, le sac comprenant un premier conduit d'entrée (11) pour l'introduction dans le sac (1) d'un fluide à pulvériser dans une cavité intracorporelle, et un second conduit de sortie (12) pour la sortie du fluide du sac (1), dans lequel le sac (1) est conformé de manière à comprendre à l'intérieur une première série de cloisons (15) qui partent d'un premier bord latéral (P1) du sac (1) et une deuxième série de cloisons (16) qui partent d'un deuxième bord latéral (P2) du sac (1), dans lequel la première série de cloisons (15) est intercalée avec la deuxième série de cloisons (16) de manière à définir, à l'intérieur du sac (1), un chemin de transport de fluide en forme de serpentine (10) qui s'étend du premier conduit d'entrée (11) au deuxième conduit de sortie (12) ;
une paire de plaques chauffantes (2, 3) qui sont configurées et agencées de manière à pouvoir recevoir entre elles le sac (1) et, lorsqu'elles sont activées, à chauffer le sac (1) et son contenu, dans lequel la paire de plaques chauffantes (2, 3) sont conformées et agencées de manière réciproque de telle sorte qu'elles définissent entre elles un espace pour le sac (1) qui permet une expansion du sac (1) lorsque celui-ci est chauffé ;
une pompe péristaltique (4) ;
dans lequel le premier conduit d'entrée (11) du sac (1) est configuré pour être raccordé à une source d'alimentation en fluide à pulvériser dans une cavité intracorporelle de manière à ce que le fluide puisse entrer dans le sac (1) et s'écouler le long du chemin de transport de fluide sous la forme d'un serpentin (10) jusqu'au deuxième conduit de sortie (12) ;
dans lequel le deuxième conduit de sortie (12) est configuré pour être raccordé à un instrument endoscopique ;
et dans lequel la pompe péristaltique (4) est agencée de manière à établir un écoulement de pompage du fluide provenant de la source d'alimentation de telle sorte que le fluide s'écoule le long du chemin de transport de fluide sous la forme d'un serpentin (10) en acquérant de la chaleur à partir de la paire de plaques chauffantes (2, 3) de manière à ce que le fluide chauffé soit pompé à travers le deuxième conduit de sortie (12) vers l'instrument endoscopique afin d'être pulvérisé dans une cavité intracorporelle.

2. Le système selon la revendication 1, dans lequel les parois de séparation de la première série de parois de séparation (15) présentes à l'intérieur du sac (1) sont disposées par rapport au premier bord latéral (P1) du sac (1) de manière à former, par rapport au premier bord latéral (P1), un angle (α) compris entre 90° et 150° et dans lequel les parois de séparation de la deuxième série de parois de séparation (16) présentes à l'intérieur du sac (1) sont disposées, par rapport au deuxième bord latéral (P2) du sac (1), de manière à former un angle compris entre 90° et 150° par rapport à la deuxième paroi latérale.

3. Le système selon l'une quelconque des revendications précédentes, dans lequel le rapport entre la distance (H) entre le premier bord latéral (P1) et le deuxième bord latéral (P2) du sac (1) et la distance (D) entre deux parois de séparation adjacentes de la première (15) et de la deuxième (16) séries de parois de séparation est compris dans la plage de 3,5 à 10.

4. Système selon l'une quelconque des revendications précédentes, dans lequel la pompe péristaltique (4) comprend un rotor (40) comportant périphériquement une série de lobes (41), et dans lequel la pompe péristaltique (4) est agencée de telle sorte que les lobes (41), lorsque le rotor (40) est activé en rotation, agissent au niveau du conduit d'entrée (11) ou du conduit de sortie (12) du sac (1) de manière à établir un écoulement de pompage du fluide provenant de la source d'alimentation de telle sorte que le fluide s'écoule le long du chemin de transport de fluide sous la forme d'un serpentin (10), en acquérant de la chaleur à partir de la paire de plaques chauffantes (2, 3) de sorte que le fluide chauffé est pompé à travers le deuxième conduit de sortie (12) vers l'instrument endoscopique afin d'être pulvérisé dans une cavité intracorporelle.

5. Système selon la revendication 4, dans lequel la pompe péristaltique (4) est agencée de telle sorte que les lobes (41), lorsque le rotor (40) est activé en rotation, agissent au niveau du conduit d'entrée (11) du sac (1) et dans lequel le système (100) comprend une chambre d'expansion (42) le long du premier conduit d'entrée (11), dans une position intercalée entre le sac (1) et la partie du conduit d'entrée (11) sur laquelle agissent les lobes (41) du rotor (40), de telle sorte que le fluide est fourni et remplit partiellement la chambre d'expansion (41) avant d'être pompé dans le sac (1) et de traverser le chemin de transport de fluide sous la forme d'un serpentin (10).

6. Le système selon l'une quelconque des revendications précédentes, dans lequel une première plaque chauffante (2) de la paire de plaques chauffantes (2, 3) est disposée et montée sur une première structure (5) ayant une forme de tiroir ou de glissière et dans lequel la deuxième plaque chauffante (3) de la paire de plaques chauffantes (2, 3) est disposée et montée sur une deuxième structure (6) ayant une forme de conteneur conformée de manière à comprendre à l'intérieur un logement, accessible de l'extérieur, ayant une forme telle qu'il reçoit de manière coulissante la première structure (5) ayant une forme de tiroir ou de glissière, de telle sorte que la première structure (5) ayant une forme de tiroir ou de glissière soit placée dans une position telle que la première plaque chauffante (2) soit disposée en face de la deuxième plaque chauffante (3) de manière à fermer entre elles le sac (1) disposé et positionné sur la première plaque chauffante (2).

7. Le système selon la revendication 6, dans lequel le sac (1) comprend des fentes respectives (20) sur les premier et deuxième bords latéraux relatifs (P1, P2) et dans lequel la première plaque chauffante (2) comprend, sur ses bords latéraux respectifs, des éléments de crochet (22) conformés de manière à être insérés dans les fentes (20) du sac (1) afin d'assurer le blocage du sac (1) sur la première plaque chauffante (2).

8. Le système selon la revendication 6, dans lequel la première structure (5) ayant une forme de tiroir ou de glissière comprend une paroi de base (51), sur laquelle la première plaque chauffante (2) est montée et disposée, et une paroi avant (52) disposée verticalement au niveau d'un premier bord latéral de la paroi de base (51), dans lequel, lorsque la première structure (5) ayant une forme de tiroir ou de glissière est insérée dans le boîtier de la deuxième structure (6) ayant une forme de récipient, la paroi avant (52) reste directement accessible de l'extérieur, et dans lequel la paroi avant (52) comprend une première ouverture (53), ayant une forme adaptée pour permettre le passage du premier conduit d'entrée (11) du sac (1) de telle sorte que le premier conduit d'entrée puisse être raccordé à la source d'alimentation en fluide, et une deuxième ouverture (54), ayant une forme adaptée pour permettre le passage du deuxième conduit de sortie (12) du sac (1) de telle sorte que le deuxième conduit de sortie puisse être raccordé à un instrument endoscopique, et dans lequel le rotor (40) de la pompe péristaltique (4) est monté sur la paroi avant (52) dans une position telle qu'il reste positionné à l'intérieur de la deuxième structure (6) en forme de récipient lorsque la première structure (5) ayant la forme d'un tiroir ou d'une glissière est insérée dans le boîtier de la deuxième structure (6) en forme de récipient.

9. Système selon l'une quelconque des revendications 6 à 8, comprenant au moins une paroi de séparation inclinée (58), disposée dans la première structure (5) en forme de tiroir ou de glissière, sous la première plaque chauffante (2), configurée pour recevoir toute fuite de fluide sortant du sac (1) et pour diriger les fuites de fluide vers un bac de collecte (59) également disposé dans la première structure (5) ayant la forme d'un tiroir ou d'une glissière.

10. Le système selon l'une quelconque des revendications précédentes, dans lequel la paire de plaques chauffantes (2, 3) comprend des détecteurs de température (20, 30) disposés et configurés pour détecter la température de chauffage du sac (1).

11. Le système selon l'une quelconque des revendications précédentes, comprenant au moins un détecteur de pression, agencé de manière à détecter la pression de pompage du flux de fluide qui traverse le chemin de transport du flux sous la forme d'un serpentin (10) du sac (1).
